# EUROPÄISCHE PATENTSCHRIFT

(11) **EP 0 589 862 B1**
(45) Veröffentlichungstag und Bekanntmachung des Hinweises auf die Patenterteilung: **27.11.1996**
(21) Anmeldenummer: 93890130.3
(22) Anmeldetag: 30.06.1993
(51) Int. Cl.: A61B 5/00, G01N 21/77

(54) **Sensoranordnung**
Sensor device
Dispositif capteur

(30) Priorität: 25.09.1992 AT 1914/92
(43) Veröffentlichungstag der Anmeldung: 30.03.1994
(73) Patentinhaber: AVL Medical Instruments AG, CH-8207 Schaffhausen (CH)
(72) Erfinder: Lübbers, Dietrich Werner, Dr., D-4600 Dortmund (DE); Karpf, Hellfried, Dr., A-8043 Graz (AT)
(74) Vertreter: Krause, Walter, Dr. Dipl.-Ing.

(56) Entgegenhaltungen:
- EP-A- 0 477 501
- DE-A- 4 001 760
- US-A- 4 741 343

## Beschreibung

Die Erfindung betrifft eine Sensoranordnung mit einem in ein Gewebe einführbaren, dünnen, biokompatiblen Röhrchen oder Schlauch, beispielsweise Mikrodialyseschlauch, welcher die zu messenden Gewebeparameter erfaßt, sowie mit einer Auswerteeinrichtung.

Aus dem Artikel "A microdialysis method allowing characterization of intercellular water space in humans", P. Lönnroth, P.-A. Jansson, U. Smith, in "Dialysis Of Human Tissue In Vivo", American Physiological Society, 1987, S. 228-231, ist es bekannt, ins Gewebe implantierbare, flexible, dünne Schläuche, z.B. zur Untersuchung des Glucosegehaltes der Zellflüssigkeit, zu verwenden. Die Wand eines solchen Mikrodialyseschlauches ist für den zu untersuchenden Stoff durchlässig, sodaß dieser in das Schlauchinnere diffundieren kann. Trotzdem ist durch die trennende Schlauchwand das Infektionsrisiko relativ gering. Zur Analyse der gesuchten Substanz kann in einem Mikrodialyseschlauch von außen ein Sensor eingeführt werden, oder der Schlauch wird mit Flüssigkeit durchströmt, die dann außerhalb analysiert wird. Diese auch größere Gewebebereiche erfassende Meßmethode hat allerdings den Nachteil, daß nur Mittelwerte der Konzentration über die gesamte benetzte Schlauchoberfläche ermittelbar, und daß lokale Schwankungen nicht festzustellen sind.

Bei vielen Eingriffen besteht in der Chirurgie das Bedürfnis, nicht nur die arteriellen Blutgase zu kontrollieren, sondern zu ermitteln, wie gut z.B. das behandelte Organ während der Operation oder hinterher durchblutet und mit Sauerstoff versorgt wird. Es hat sich gezeigt, daß es durch Messung des lokalen Sauerstoffdruckes im Gewebe möglich ist, den Status der Sauerstoffversorgung zu ermitteln. Als Methode hierfür ist z.B. die polarographische Messung eines pO₂-Histogrammes mit einer dünnen membranbedeckten Nadelelektrode möglich. Ein solches pO₂-Histogramm besteht aus einer größeren Anzahl an verschiedenen Gewebestellen gemessenen Einzelwerten. Da diese Methode aber nicht zur kontinuierlichen Überwachung geeignet ist, werden kontinuierlich messende polarographische pO₂-Katheterelektroden ins Gewebe eingeführt, z.B. Katheterelektroden mit geringem Außendurchmesser von ca. 0.55 mm. Der Nachteil dieser Elektroden ist, daß sie den pO₂ nur an einer einzigen Stelle des Gewebes erfassen, von der man nicht weiß, wie gut sie die Gesamtsituation charakterisiert.

Ein weiterer Nachteil ist, daß es mit solchen polarographischen Elektroden nur möglich ist, relative pO₂-Werte zu messen. Die Eichkurve, die in Gas- oder Salzlösungen erhoben wird, läßt sich nämlich nicht auf Messungen im Gewebe übertragen, da die Parameter, die die Steilheit der Eichkurve bestimmen, im Gewebe außerordentlich variieren und daher nicht mit Sicherheit vorhersagbar sind.

Weiters wurden beispielsweise aus der AT-B 392 539 optische Sensoren mit einem Lichtleiter und einer darauf immobilisierten Indikatorsubstanz bekannt, welche allerdings nicht zur Messung von Konzentrationsprofilen im Gewebe geeignet sind. Daraus geht ein Sensor zur optischen Bestimmung der katalytischen Enzymaktivität einer Probe hervor, bei welchem auf der äußeren Fläche des zylinderförmigen Kerns eines Lichtleiters ein Enzymsubstrat angebracht ist. Beim Messen wird die Spitze des Sensors in die Probenlösung eingetaucht.

Aufgabe der Erfindung ist es, eine Sensoranordnung vorzuschlagen, mit welcher zur Erfassung der Heterogenität des Gewebes gleichzeitig an mehreren Stellen des Gewebes die lokale Konzentration einer bestimmten Spezies oder die Konzentration unterschiedlicher Analyte kontinuierlich erfaßt werden kann.

Diese Aufgabe wird erfindungsgemäß dadurch gelöst, daß der Schlauch in axialer Erstreckung mehrere Bereiche aufweist, in welchen jeweils gleiche oder unterschiedliche, optisch anregbare und ablesbare, vorzugsweise lumineszenzoptische, Indikatorsubstanzen zur Messung der örtlichen Verteilung eines Parameters, beispielsweise der O₂-Konzentration bzw. des O₂-Partialdruckes oder zur gleichzeitigen Messung zumindest zweier unterschiedlicher Parameter, beispielsweise aus der Gruppe pO₂, pCO₂ und pH, vorgesehen sind. Unter Schlauch soll auch ein Schlauchsystem verstanden werden, welches flexible und feste Anteile aufweist, welche Anteile die für die jeweiligen Einsatzgebiete nötige Festigkeit, Permeabilität und Transparenz aufweisen. Die Indikatoren können ringförmig am Schlauchumfang oder auch lediglich an einigen Stellen an der Schlauchoberfläche angebracht oder in den Schlauch integriert sein. Die optische Ablesung von auperhalb des Schlauches anliegenden Indikatoren bedingt lediglich die Lichtleitfähigkeit des Schlauchmaterials. Die Schlauchwand kann für den (die) Analyt(e) undurchlässig sein, aber andere Stoffe durchlassen. Ist der Indikator im Schlauchinneren vorgesehen oder in den Schlauch integriert, muß die Schlauchwand zusätzlich für den (die) Analyt(e) durchlässig sein. Mit Hilfe dieser Sensoranordnung können viele Meßpunkte im Gewebe mit einer einzigen Meßleitung erfaßt werden, wodurch das Gewebetrauma minimiert wird. Als Indikatorsubstanzen sind sowohl Absorptions- oder Lumineszenz, als auch Chemilumineszenz-Indikatoren verwendbar.

Um mit dieser Sensoranordnung die Absolutwerte einer Analytkonzentration gleichzeitig an mehreren Stellen bzw. Tiefenlagen des Gewebes zu bestimmen, sind beispielsweise unterschiedliche Indikatorsubstanzen zur Messung einer Spezies vorgesehen. Durch Isolation der Signale kann auf diese Weise das Konzentrationsprofil bzw. die örtliche Verteilung eines Analytes ermittelt werden. Damit kann die Heterogenität des Gewebes kontinuierlich erfaßt werden. Indikatorsysteme können sich dabei aus gleichen oder unterschiedlichen Indikatorsubstanzen oder aus Enzym-Indikatorsubstanz-Kombinationen zusammensetzen.

Weiters können in den einzelnen Bereichen auch unterschiedliche Indikatorsubstanzen für die gleichzeitige Messung zumindest zweier Konzentrationswerte z.B. aus der Gruppe pO₂, pCO₂ und pH vorgesehen sein. Es kann auch eine Kombination von Indikatorsubstanzen in oder auf dem biokompatiblen Schlauch angebracht werden. Eine Kombination von pO₂- und pH-Indikatorsubstanzen kann von Vorteil sein, da beispielsweise bei verschlechteter Sauerstoffversorgung auch der pH-Wert sinkt. Mit geeigneten Indikatorsystemem können auch andere interessierende Stoffe im Gewebe, wie zum Beispiel Kalium, Kalzium, Glucose, Hormone oder Narkosegase gemessen werden. Die in Abhängigkeit von der Analytkonzentration von den einzelnen Indikatorsubstanzen abgegebenen Signale lassen sich optisch, z.B. durch geeignete Filterung, isolieren und getrennt verarbeiten.

Es ist auch eine Kombination mit anderen Sensoren möglich. So kann z.B. die Messung der innergeweblichen Temperatur, des Druckes, der elektrischen Aktivität, der Radioaktivität und der Wasserstoffclearance wichtige Zusatzinformationen liefern.

Bei den erfindungsgemäßen Ausführungsvarianten des Sensors, bei welchem das Anregungslicht im Lichtleiter mittels Totalreflexion geführt wird, müssen entweder Maßnahmen gesetzt werden, die das Anregungslicht im Bereich der Indikatorsubstanzen austreten lassen oder die Anregung erfolgt durch die evaneszenten Wellenanteile des Anregungslichtes, wie beispielsweise aus der US-A 3 604 927 bekannt.

Ein Übertritt der Anregungsstrahlung vom optischen Leiter in die Indikatorschicht kann auch durch die geeignete Wahl des Brechungsindex der Indikatorschicht gewährleistet werden (DE-C2 35 32 563).

In einer vorteilhaften Ausführungsvariante ist vorgesehen, daß der Schlauch eine als Lichtleiter ausgebildete Schlauchwand aufweist. Damit wird auf einfache Weise eine gleichzeitige lokale optische Messung in der Schlauchwand, und eine Mittelwertmessung der in das Innere diffundierenden Analyte möglich. Eine derartige gleichzeitige lokale und globale Messung ist auch möglich, wenn der Brechungsindex der Schlauchwand kleiner ist als jener einer flüssigen, gelartigen oder festen Schlauchfüllung, sodaß die Lichtleitung in der Schlauchfüllung erfolgt. Die in die flüssige Schlauchfüllung diffundierenden Analyte können nach außen geführt und auf herkömmliche Weise analysiert werden.

Falls die nötige Festigkeit zum Einstechen in das Gewebe nicht vorhanden ist, kann der Schlauch erfindungsgemäß von einer nach dem Einführen in das Gewebe entfernbaren Hohlnadel umgeben sein.

In einer weiteren vorteilhaften Ausführungsvariante der Erfindung ist vorgesehen, daß das im Gewebe liegende eine Ende des Schlauches verspiegelt ist und das andere Ende in an sich bekannter Weise für die Einkopplung der Anregungsstrahlung und die Gewinnung der Meßstrahlung zur Verfügung steht.

In Ausgestaltung der Erfindung ist vorgesehen, daß der Schlauch durch das Gewebe durchführbar ist, sodap beide Enden aus der Gewebeoberfläche ragen, wobei z.B. ein Ende für die Einkopplung der Anregungsstrahlung und das andere Ende für die Gewinnung der Meßstrahlung zur Verfügung steht. Andererseits ist es auch denkbar, ein Ende des Schlauches sowohl zum Einbringen der Anregungsstrahlung, als auch zum Gewinnen der Meßstrahlung zu verwenden, und das andere Ende für weitere medizinische Zwecke zu nutzen, beispielsweise zur Zufuhr von Substanzen um eine Testreaktion auszulösen.

Für die Messung der Oberflächenkonzentration einer bestimmten Spezies kann erfindungsgemäß um zumindest einem freien Ende des Schlauches eine an der Gewebeoberfläche anliegende Optodenfolie angeordnet sein.

Eine weitere vorteilhafte Ausgestaltung der Erfindung besteht darin, daß als Lichtleiter eine in axialer Richtung in den Schlauch einführbare Sonde mit einer oder mehreren gegen die Längsachse um etwa 45° geneigten spiegelnden Flächen, vorzugsweise dichroitischen Spiegel(n), vorgesehen ist. Die Ablesung der Indikatorsubstanzen kann mittels der Sonde automatisch erfolgen, wobei die Sonde durch eine Ziehvorrichtung oder Spindel positioniert wird.

Für Messungen im Knochengewebe oder im Gelenkbereich kann beispielsweise ein Stahlröhrchen oder eine Kanüle mit Löchern in der Wandung eingesetzt werden. Die in den Löchern angeordneten Sensoren bzw. Indikatorsubstanzen können ebenfalls mit einer in die Kanüle einführbaren Sonde abgelesen werden.

In einer bevorzugten Ausführungsvariante ist vorgesehen, daß der Schlauch, in zumindest einem Bereich, transparent ausgebildet ist. Dadurch können Gewebebeobachtungen durchgeführt und auch außerhalb des Schlauches vorhandene Indikatoren optisch erfaßt werden. Solche im Gewebe vorliegende Indikatoren sind beispielsweise der Blutfarbstoff (Hämoglobin), der Muskelfarbstoff (Myoglobin) oder das fluoreszierende Enzym NADH der Atmungskette.

Der in das Gewebe eingebrachte optische Leiter muß nicht in direktem Kontakt mit einer optischen Anregungs- und Auswerteeinheit stehen, vielmehr kann erfindungsgemäß eine an sich bekannte Einrichtung zur berührungslosen Einkopplung der Anregungsstrahlung und Gewinnung der Meßstrahlung vorgesehen sein, welche die Auswerteeinrichtung enthält.

Die berührungslose Messung optischer Signale auf sich bewegenden Oberflächen ist aus "Oxygen Transport To Tissue VIII", N. Opitz und D.W. Lübbers, Adv.Exp.Med.Biol. 200, 367-371, Plenum Press, 1986 bekannt.

Da menschliches und tierisches Gewebe, insbesondere die Haut, für optische Strahlen im roten und infraroten Wellenlängenbereich relativ gut durchlässig ist, kann erfindungsgemäß auch eine Indikatorsubstanz zur Anwendung kommen, deren Anregungs- und Emissionsstrahlung im Wellenlängenbereich zwischen ca. 600 nm und 1200 nm liegt. In diesem Fall wird der Sensor von außen angeregt und abgelesen, z.B. durch Sensoren oder Sensorareale auf der Hautoberfläche.

Zur Verbesserung der Gewebeanalyse ist es vorteilhaft, wenn der Schlauch, zumindest teilweise, aus einem die zu messende Substanz anreichernden Material besteht. Vom Material Silikon ist beispielsweise bekannt, daß es verschiedene Stoffe wie CO₂ oder Narkosegase anreichert, wodurch die genaue Messung auch geringer Konzentrationen dieser Substanzen ermöglicht wird.

Die Erfindung wird im folgenden anhand von Ausführungsbeispielen näher erläutert. Es zeigen:
- Fig. 1: eine Sensoranordnung gemäß der Erfindung und die
- Fig. 2 und 3: erfindungsgemäße Ausführungsvarianten,
- Fig. 4 bis 9: Detaildarstellungen von Mikrodialyseschläuchen mit unterschiedlicher Signalübertragung gemäß der Erfindung.

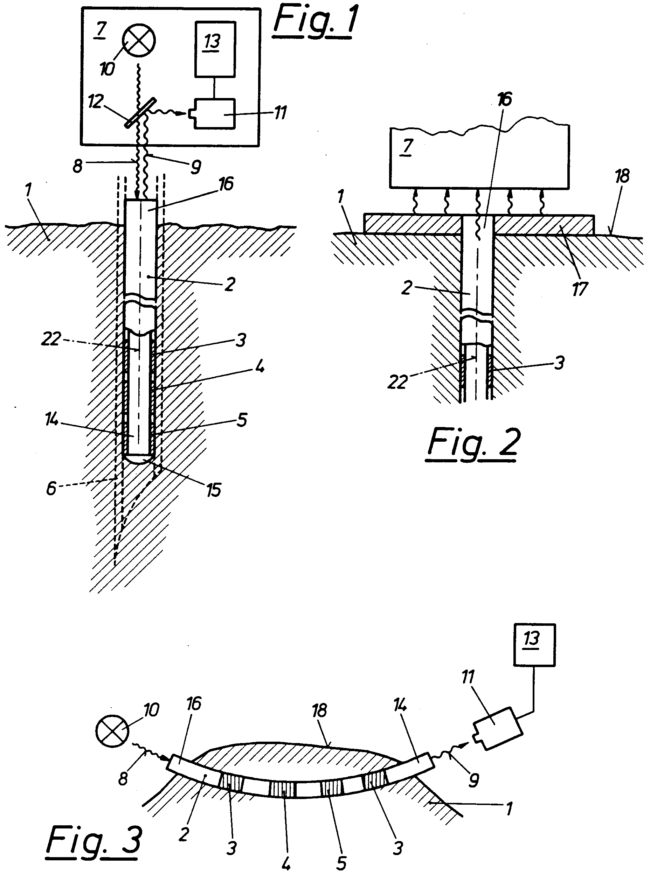

In Fig. 1 ist eine Sensoranordnung zur Messung der Konzentrationsverteilung im Gewebe dargestellt, welche auf dem ins Gewebe 1 einführbaren, dünnen, biokompatiblen Schlauch 2 (bzw. Kanüle) in axialer Erstreckung mehrere Bereiche 3 bis 5 aufweist, in welchen Indikatorsubstanzen mit unterschiedlichen optischen Eigenschaften immobilisiert sind. Falls der Schlauch 2, beispielsweise ein Mikrodialyseschlauch, selbst nicht die nötige Rigidität aufweist, kann eine nach dem Einstechen entfernbare Hohlnadel 6 für das Einführen in das Gewebe vorgesehen sein. Der Schlauch 2 des Sensors kann direkt mit einer Lichtquelle und einem Detektor verbunden sein, oder - wie in Fig. 1 dargestellt - über eine Einrichtung 7 zur berührungslosen Einkopplung der Anregungsstrahlung 8 und Gewinnung der Meßstrahlung 9 verfügen. In der Einrichtung 7 sind neben einer Strahlungsquelle 10 und einem Detektor 11 ein Strahlteiler 12 zur Abtrennung der Meßstrahlung von der Anregungsstrahlung und eine mit dem Detektor 11 verbundene Auswerteeinrichtung 13 vorgesehen. Bei dieser Ausführungsvariante ist das im Gewebe 1 liegende Ende 14 des Schlauchs 2 mit einer verspiegelten Kappe 15 versehen und das freie Ende 16 der Einkoppeleinrichtung 7 zugekehrt.

Fig. 2 zeigt eine Variante des Sensors nach Fig. 1, welche rings um das freie Ende 16 des Schlauches 2 eine Optodenfolie 17 aufweist, welche an der Gewebeoberfläche 18 aufliegt und die Flächenkonzentration einer Spezies (z.B. die pO₂-Verteilung) mißt.

In Fig. 3 ist eine Ausführungsvariante gezeigt, bei welcher der Schlauch 2 durch das Gewebe 1 durchgeführt ist, sodap ein Ende 14 für die Einkoppelung der Anregungsstrahlung 8 und das andere Ende 16 für die Gewinnung der Meßstrahlung 9 zur Verfügung steht.

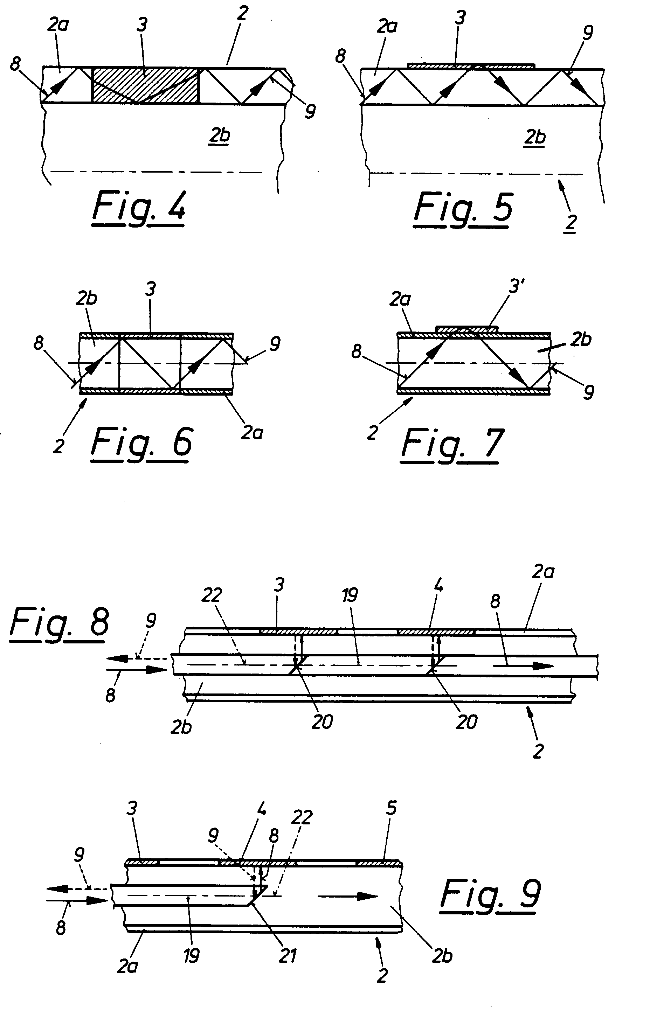

In Fig. 4 und 5 erfolgt die Lichtleitung ausschließlich in der Schlauchwand 2a. Die Indikatorsubstanzen sind dabei entweder in die Schlauchwand 2a integriert und werden durch die Anregungsstrahlen 8 bzw. Meßstrahlen 9 durchdrungen (Fig. 4), oder sie sind auf die Schlauchwand 2a aufgearbeitet und reflektieren die Anregungsstrahlung 8 bzw. Meßstrahlung 9 (Fig. 5).

Durch passende Abstimmung der Brechindizes von Schlauchwand 2a und Schlauchfüllung 2b aufeinander, kann das Licht überwiegend in der Schlauchfüllung 2b geleitet werden, wie dies in Fig. 6 und 7 dargestellt ist. Die Indikatorsubstanz eines Bereiches 3 kann wieder entweder in die Schlauchwand 3 integriert (Fig. 6), oder auf die Oberfläche der Schlauchwand aufgesetzt sein (Fig. 7).

Die Übertragung der Lichtsignale kann aber ebenso über eine, in den Schlauch 2 einführbare Sonde 19 erfolgen, wie aus Fig. 8 ersichtlich ist. Zur Ablesung der Indikatorsubstanzen sind mehrere auf der Sonde 19 hintereinander angeordnete dichroitische Spiegel 20 vorgesehen. In einer Stellung der Sonde 19 können dabei mehrere Indikatorsubstanzen gleichzeitig abgelesen werden.

Für Anwendungen im Knochengewebe oder im Gelenkbereich ist es auch möglich eine Kanüle zu verwenden, welche in den Bereichen 3 bis 5 Löcher aufweist, in welchen die Indikatorsubstanzen angeordnet sind.

Wie in Fig. 9 gezeigt, kann die Ablesung der Indikatorsubstanzen auch nacheinander erfolgen, wobei auf der Sonde 19 nur eine einzige spiegelnde Fläche 21 vorgesehen ist. Dabei muß der Spiegel 21 im Bereich der jeweils abzulesenden Indikatorsubstanz positioniert werden. Nach Übertragung der Daten wird die Sonde in axialer Richtung zum nächsten, eine weitere Indikatorsubstanz aufweisenden Bereich 3, 4 oder 5 geführt.

Neben der Verwendung zur Gewebeanalyse läßt sich die gezeigte Sensoranordnung auch auf anderen technischen oder naturwissenschaftlichen Anwendungsgebieten, beispielsweise zur Überwachung eines Bioreaktors oder zur Bestimmung der Gute eines Gewässers, vorteilhaft einsetzen.

## Patentansprüche

1. Sensoranordnung mit einem in ein Gewebe einführbaren, dünnen, biokompatiblen Röhrchen oder Schlauch, beispielsweise Mikrodialyseschlauch, welcher die zu messenden Gewebeparameter erfaßt, sowie mit einer Auswerteeinrichtung, **dadurch gekennzeichnet**, daß der Schlauch (2) in axialer Erstreckung mehrere Bereiche (3, 4, 5...) aufweist, in welchen jeweils gleiche oder unterschiedliche, optisch anregbare und ablesbare, vorzugsweise lumineszenoptische, Indikatorsubstanzen zur Messung der örtlichen Verteilung eines Parameters, beispielsweise der O₂-Konzentration bzw. des O₂-Partialdruckes oder zur gleichzeitigen Messung zumindest zweier unterschiedlicher Parameter, beispielsweise aus der Gruppe pO₂, pCO₂ und pH, vorgesehen sind.

2. Sensoranordnung nach Anspruch 1, **dadurch gekennzeichnet**, daß der Schlauch (2) eine als Lichtleiter ausgebildete Schlauchwand (2a) aufweist.

3. Sensoranordnung nach Anspruch 1, **dadurch gekennzeichnet**, daß der Brechungsindex der Schlauchwand (2a) kleiner ist als jener einer flussigen, gelartigen oder festen Schlauchfüllung (2b), sodaß die Lichtleitung in der Schlauchfüllung (2b) erfolgt.

4. Sensoranordnung nach einem der Ansprüche 1 bis 3, **dadurch gekennzeichnet,** daß der Schlauch (2) von einer nach dem Einführen in das Gewebe (1) entfernbaren Hohlnadel (6) umgeben ist.

5. Sensoranordnung nach einem der Ansprüche 1 bis 4, **dadurch gekennzeichnet**, daß das im Gewebe liegende eine Ende (14) des Schlauches (2) verspiegelt ist und das andere Ende (16) in an sich bekannter Weise für die Einkopplung der Anregungsstrahlung (8) und die Gewinnung der Meßstrahlung (9) zur Verfügung steht.

6. Sensoranordnung nach einem der Ansprüche 1 bis 4, **dadurch gekennzeichnet**, daß der Schlauch (2) durch das Gewebe (1) durchführbar ist, sodaß beide Enden (14, 16) aus der Gewebeoberfläche (18) ragen, wobei z.B. ein Ende (14) für die Einkopplung der Anregungsstrahlung (8) und das andere Ende (16) für die Gewinnung der Meßstrahlung (9) zur Verfügung steht.

7. Sensoranordnung nach einem der Ansprüche 1 bis 5, **dadurch gekennzeichnet**, daß um zumindest einem freien Ende (16) des Schlauches (2) eine an der Gewebeoberfläche (18) anliegende Optodenfolie (17) zur Messung der Oberflächenkonzentration einer bestimmten Spezies angeordnet ist.

8. Sensoranordnung nach einem der Ansprüche 1 bis 4, **dadurch gekennzeichnet**, daß als Lichtleiter eine in axialer Richtung in den Schlauch (2) einführbare Sonde (19) mit einer oder mehreren gegen die Längsachse (22) um etwa 45° geneigten spiegelnden Fläche(n) (21), vorzugsweise dichroitischen Spiegel(n) (20), vorgesehen ist.

9. Sensoranordnung nach einem der Ansprüche 1 bis 8, **dadurch gekennzeichnet**, daß der Schlauch (2), in zumindest einem Bereich, transparent ausgebildet ist.

10. Sensoranordnung nach einem der Ansprüche 1 bis 9, **dadurch gekennzeichnet,** daß eine an sich bekannte Einrichtung (7) zur berührungslosen Einkoppelung der Anregungsstrahlung (8) und Gewinnung der Meßstrahlung (9) vorgesehen ist, welche die Auswerteeinrichtung (13) enthält.

11. Sensoranordnung nach Anspruch 1, **dadurch gekennzeichnet**, daß eine Indikatorsubstanz zur Anwendung kommt, deren Anregungs-und Emissionsstrahlung im Wellenlängenbereich zwischen ca. 600 nm und 1200 nm liegt.

12. Sensoranordnung nach einem der Ansprüche 1 bis 11, **dadurch gekennzeichnet,** daß der Schlauch (2), zumindest teilweise, aus einem die zu messende Substanz anreichernden Material besteht.

## Claims

1. A sensing device comprising a thin biocompatible cannula or tube, such as a microdialysis tube, which may be inserted into human or animal tissue and measures the tissue parameters of interest, and further comprising an evaluation unit, **wherein** the tube (2) has several areas (3, 4, 5, ...) along its length, which contain either identical or different, optically excitable and readable, preferably luminescence-optical indicating substances for measuring the local distribution of one parameter, such as O₂ concentration or O₂ partial pressure, or for simultaneous measurement of at least two different parameters, belonging to the pO₂, pCO₂, pH group, for example.

2. A sensing device as in claim 1, **wherein** the tube (2) has a tube wall (2a) which is configured as a light guide.

3. A sensing device as in claim 1, **wherein** the refractive index of the tube wall (2a) is lower than that of a liquid, gel-like or solid tube filling (2b), so that the light is guided in the tube filling (2b).

4. A sensing device as in any of claims 1 to 3, **wherein** the tube (2) is surrounded by a hollow needle (6), which can be removed after the tube (2) is inserted in the tissue (1).

5. A sensing device as in any of claims 1 to 4, **wherein** the end (14) of the tube (2) located in the tissue is mirror-faced and the other end (16) is used for feeding in the excitation radiation (8) and picking up the measurement radiation (9) in a known manner.

6. A sensing device as in any of claims 1 to 4, **wherein** the tube (2) is threaded through the tissue (1) so that both ends (14, 16) project from the tissue surface (18), one end (14) being available for feeding in the excitation radiation (8) and the other one (16) for picking up the measurement radiation (9).

7. A sensing device as in any of claims 1 to 5, **wherein** for the purpose of measuring the surface concentration of a given species at least one (16) of the free ends of the tube (2) is provided with an optode foil (17) in contact with the tissue surface (18).

8. A sensing device as in any of claims 1 to 4, **wherein** the light guide is configured as a probe (19) with one or more mirror faces (21) inclined towards the longitudinal axis (22) by about 45°, i.e., preferably dichroitic mirrors (20), the probe (19) being inserted into the tube (2) in axial direction.

9. A sensing device as in any of claims 1 to 8, **wherein** the tube (2) is transparent in at least one area.

10. A sensing device as in any of claims 1 to 9, **wherein** a known device (7) is used for contact-free feeding of the excitation radiation (8) and pick-up of the measurement radiation (9), which includes the evaluation unit (13).

11. A sensing device as in claim 1, **wherein** an indicator substance is used whose excitation/emission radiation has a wavelength of 600 to 1,200 nm, approximately.

12. A sensing device as in any of claims 1 to 11, **wherein** the tube (2), at least in parts, is made of material accumulating the sample substance.

## Revendications

1. Dispositif de détection comportant un petit tube ou un tuyau mince, biocompatible, pouvant être introduit dans un tissu organique, par exemple un tuyau de microdialyse opérant la saisie des paramètres physiologiques à mesurer, aussi qu'un dispositif d'exploitation,
caractérisé en ce que
le tuyau (2) présente, selon sa direction axiale, plusieurs zones (3, 4, 5...) dans lesquelles se trouvent des substances indicatrices, identiques ou différentes, pouvant être excitées optiquement et lues, de préférence, par voie lumineuse, ces substances servant à mesurer la distribution locale d'un paramètre, par exemple la concentration en oxygène ou sa pression partielle, ou à mesurer en même temps au moins deux paramètres différents, par exemple du groupe pO₂, pCO₂ et pH.

2. Dispositif de détection selon la revendication 1,
caractérisé en ce que
le tuyau (2) a une paroi (2a) constituée par un guide de lumière ou fibre optique.

3. Dispositif de détection selon la revendication 1,
caractérisé en ce que
l'indice de réfraction de la paroi (2c) est inférieur à celui du remplissage (2b) de chacun d'un tuyau, liquide, en gel, ou solide, de sorte que la transmission de lumière a lieu à l'intérieur du remplissage (2b).

4. Dispositif de détection selon une des revendications 1 à 3,
caractérisé en ce que
le tuyau (2) est entouré par une aiguille creuse (6) qui peut être retirée après l'introduction dans le tissu (1).

5. Dispositif de détection selon une des revendications 1 à 4,
caractérisé en ce que
l'extrémité (14) du tube (2) qui se trouve dans le tissu est réfléchissante tandis que l'autre extrémité (16) est disponible de manière connue, pour injecter le rayonnement d'excitation (8) et recueillir le rayonnement de mesure (9).

6. Dispositif de détection selon une des revendications 1 à 4,
caractérisé en ce que
le tuyau (2) peut être passé à travers le tissu (1), de manière que ses deux extrémités (14, 16) dépassent au dessus de la surface (18) du tissu, une de ces extrémités (14), par exemple, pouvant servir à injecter le rayonnement d'excitation (8) tandis que l'autre extrémité (16) peut servir à recueillir le rayonnement de mesure (9).

7. Dispositif de détection selon une des revendications 1 à 5,
caractérise en ce qu'
il est prévu, autour d'au moins une extrémité libre (16) du tuyau (2) une feuille optique (17) placée à la surface (18) du tissu et servant à mesurer la concentration superficielle d'une substance chimique donnée.

8. Dispositif de détection selon une des revendications 1 à 4,
caractérisé en ce que
les guide de lumière est constitué par une sonde (19) qui peut être introduite dans le tuyau (2) et qui est équipée d'une ou plusieurs surfaces réfléchissantes (21), inclinées à 45 degrés environ par rapport à l'axe longitudinal (22) et constituées de préférence par des miroirs dichroïdes (20).

9. Dispositif de détection selon une des revendications 1 à 8,
caractérisé en ce que
le tuyau (2) est, au moins dans une certaine zone, transparent.

10. Dispositif de détection selon une des revendications 1 à 9,
caractérisé en ce qu'
il comprend un dispositif (7) connu en soi, assurant une injection sans contact du rayonnement d'excitation (8) ainsi que la réception de rayonnement de mesure (9) qui comprend le dispositif d'exploitation (13).

11. Dispositif de détection selon la revendication 1, caractérisé en ce qu'
il utilise, comme substance indicatrice, une substance dont le rayonnement d'excitation et le rayonnement d'émission se situent dans la plage des longueurs d'ondes s'étendant entre 600 et 1 200 nm environ.

12. Dispositif de détection selon une des revendications 1 à 11,
caractérisé en ce que
le tuyau (2) est fait, au moins partiellement, d'un matériau enrichissant la substance chimique à mesurer.
